(19) Europäisches Patentamt
European Patent Office
Office européen des brevets

(11) Veröffentlichungsnummer: **0 253 767**
**A1**

# (12) EUROPÄISCHE PATENTANMELDUNG

(21) Anmeldenummer: **87810370.4**

(22) Anmeldetag: **26.06.87**

(51) Int. Cl.[4]: **C 07 D 493/22**
C 07 F 7/08, A 01 N 43/90
//(C07D493/22,313:00,311:00,
311:00,307:00)

(30) Priorität: **02.07.86 CH 2669/86**

(43) Veröffentlichungstag der Anmeldung:
**20.01.88 Patentblatt 88/03**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Frei, Bruno, Dr.**
**Bündtenstrasse 16**
**CH-4410 Liestal (CH)**

(54) **Parasitizide und Insektizide.**

(57) Es werden parasitizid und insektizid hochaktive 13β-Oxacycloalkylcarbonyl-Milbemycine der Formel I

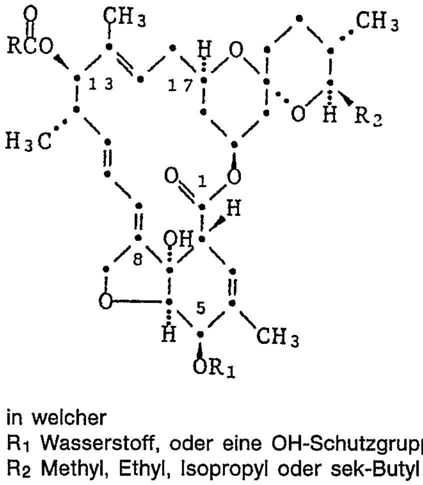

(I)

in welcher
$R_1$ Wasserstoff, oder eine OH-Schutzgruppe,
$R_2$ Methyl, Ethyl, Isopropyl oder sek-Butyl und
R für einen Oxacycloalkylrest steht,
sowie deren Herstellung und deren Anwendung beschrieben.

EP 0 253 767 A1

**Beschreibung**

Parasitizide und Insektizide

Die vorliegende Erfindung betrifft neue 13β-Oxacycloalkyl-carbonyloxy-milbemycin-Derivate der Formel I, ihre Herstellung und ihre Verwendung zur Bekämpfung von Schädlingen, ferner Schädlingsbekämpfungsmittel, die mindestens eine dieser Verbindungen als Wirkstoff enthalten.

Die neuen Verbindungen entsprechen der allgemeinen Formel I

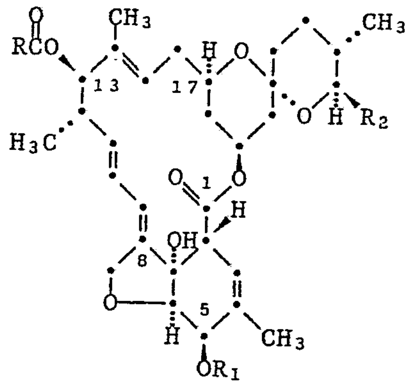

(I)

in welcher

$R_1$ Wasserstoff, oder eine OH-Schutzgruppe,

$R_2$ Methyl, Ethyl, Isopropyl oder sek.Butyl und

R für einen Oxacycloalkylrest steht.

Unter OH-Schutzgruppen für den Substituenten $R_1$ sollen hier und im folgenden die in der organischen Chemie üblichen Schutzfunktionen verstanden werden. Dabei handelt es sich insbesondere um Acyl- und Silylgruppen. Geeignete Acylgruppen sind beispielsweise die Reste $R_3$-C(O)-, vorzugsweise für $C_1$-$C_6$-Alkyl, $C_1$-$C_6$-Haloalkyl oder unsubstituiertes oder durch Halogen, $C_1$-$C_3$-Alkyl, $CF_3$ oder Nitro substituiertes Phenyl steht. Als geeignete Silylgruppen für $R_1$ kommt der Rest -Si($R_4$)($R_5$)($R_6$) in Frage, wobei $R_4$, $R_5$ und $R_6$ vorzugsweise unabhängig voneinander für $C_1$-$C_4$-Alkyl, Benzyl oder Phenyl stehen und beispielsweise eine der Gruppen Trimethylsilyl, tris(tert.Butyl)silyl, Diphenyl-tert.butylsilyl, bis(Isopropyl)methylsilyl, Dimethyl-(2,3-dimethyl-2-butyl)silyl, Triphenylsilyl usw. und insbesondere tert.Butyl-dimethylsilyl bildet. Die 5-OH-Gruppe kann auch als Benzylether oder Methoxiethoximethylether vorliegen.

Verbindungen der Formel I, worin $R_1$ eine Schutzgruppe darstellt, lassen sich durch einfache, z.B. hydrolytische Abspaltung der Schutzfunktion in die hochaktiven freien 5-Hydroxi-Derivate ($R_1$ = H) überführen und haben somit Zwischenprodukte-Charakter. Im übrigen wird der biologische Wert dieser Verbindungen durch die Schutzgruppe im Prinzip nicht gemindert.

Verbindungen, worin $R_2$ sek.Butyl darstellt, sollen hier und im folgenden gleichfalls zu den Milbemycin-Derivaten gerechnet werden, obwohl sie nach der üblichen Systematik von Avermectin-Derivaten abgeleitet sind. Avermectin-Aglykone (mit einer OH-Gruppe in 13α-Position) lassen sich jedoch gemäss US-PS 4,173,571 in Milbemycin-Homologe überführen.

In natürlich vorkommenden Milbemycinen ($R_1$ = H; $R_2$ = $CH_3$, $C_2H_5$ oder iso $C_3H_7$) ist die 13-Position anstelle der Estergruppe der erfindungsgemässen Verbindungen der Formel I ausschliesslich mit Wasserstoff besetzt, wie die nachstehende Formel zeigt:

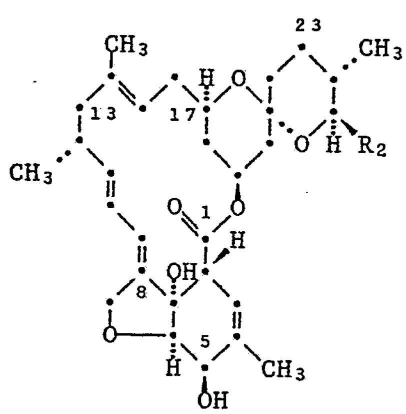

$R_2 = CH_3$ Milbemycin $A_3$
$R_2 = C_2H_5$ Milbemycin $A_4$
$R_2$ = iso$C_3H_7$ Milbemycin D
$R_2$ = sec.$C_4H_9$ 13-Deoxi-22,23-dihydro-C-076-Bla-aglycon.

Bei Avermectinen dagegen steht in der 13-Position ein α-L-Oleandrosyl-α-L-oleandrose-Rest, der über Sauerstoff in α-Konfiguration mit dem Makrolid-Molekül verknüpft ist. Avermectine unterscheiden sich strukturell ausserdem durch eine 23-OH-Gruppe oder $\Delta^{22,23}$-Doppelbindung und in der Regel durch einen Substituenten $R_2$ = sek.$C_4H_9$ von den Milbemycinen. Durch Hydrolyse des Zucker-Restes der Avermectine gelangt man leicht zu den entsprechenden Avermectin-Aglykonen, die eine in Nachbarstellung zu einer C=C-Doppelbindung befindliche 13α-Hydroxy-Gruppe besitzen. Die Avermectin-Aglykone sind, wie vorstehend angegeben, in die Milbemycin-Homologen umwandelbar. Bei den Milbemycin-Derivaten der vorliegenden Anmeldung liegt die $\Delta^{22,23}$-Doppelbindung stets in hydrierter Form vor und der Substituent in 13-Position ist immer β-ständig.

Formel I repräsentiert somit Milbemycin-Abkömmlinge, die in 5-Position entweder eine freie OH-Gruppe, eine Silyl- oder Acylgruppe aufweisen und in 13β-Position einen Oxacycloalkancarbonyloxy-Substituenten (R-COO) tragen.

Unter einem Oxacycloalkylrest R soll im Rahmen der vorliegenden Erfindung vorzugsweise der Rest der Formel U verstanden werden

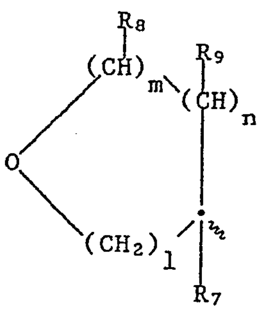

(U)

wobei 1, m und n unabhängig voneinander 0, 1, 2 oder 3 bedeuten, $R_7$, $R_8$ und $R_9$ unabhängig voneinander für Wasserstoff, Alkyl oder Phenyl stehen, oder gegebenenfalls auch eine endocyclische Doppelbindung vorliegt.

Unter dem Begriff Alkyl selbst oder als Bestandteil eines anderen Substituenten sind je nach Zahl der angegebenen Kohlenstoffatome beispielsweise folgenden Gruppen zu verstehen: Methyl, Ethyl, Propyl, Butyl, Pentyl, Hexyl, Heptyl, Octyl, Nonyl, Decyl, usw. sowie die Isomeren, wie z.B. Isopropyl, Isobutyl, tert.-Butyl, Isopentyl usw..

Als Oxacycloalkylreste kommen somit z.B. folgende Reste in Frage:
3-Methyl-3-oxetanyl, 3-Ethyl-3-oxetanyl, 3-Pentyl-3-oxetanyl, 3-Tetrahydrofuranyl, 2-Tetrahydrofuranyl, 2-Methyl-2-tetrahydrofuranyl, 2-Ethyl-2-tetrahydrofuranyl, 2-Pentyl-2-tetrahydrofuranyl, 2-Phenyl-2-tetrahydrofuranyl, 2,3-Dimethyl-2-tetrahydrofuranyl, 2,5-Dimethyl-2-tetrahydrofuranyl, 3-Ethyl-2-methyl-2-tetrahydrofuranyl, 2-Tetrahydropyranyl, 3-Tetrahydropyranyl, 2-Methyl-2-tetrahydropyranyl, 2-Ethyl-2-tetrahydropyranyl, 2-Propyl-2-tetrahydropyranyl, 2-Butyl-2-tetrahydropyranyl, 2-Pentyl-2-tetrahydropyranyl, 2-Phenyl-2-tetrahydropyranyl, 2,6-Dimethyl-2-tetrahydropyranyl, 2,6,6-Trimethyl-2-tetrahydropyranyl, 3,4-Dihydro-2H-pyranyl.

Folgende Untergruppen von Verbindungen der Formel I sind auf Grund ihrer ausgeprägten parasitiziden und insektiziden Wirkung besonders bevorzugt:

Gruppe Ia: Verbindungen der Formel I, worin R für einen Oxacycloalkylrest der Formel U steht

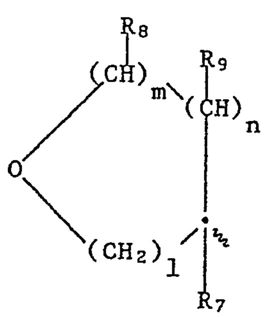

(U)

wobei 1, m und n unabhängig voneinander 0, 1, 2 oder 3 bedeuten, $R_7$, $R_8$ und $R_9$ unabhängig voneinander für Wasserstoff, Alkyl oder Phenyl stehen, gegebenenfalls auch eine endocyclische Doppelbindung vorliegt; $R_1$ Wasserstoff oder eine Schutzgruppe bedeutet; und $R_2$ für Methyl, Ethyl, Isopropyl oder sek.-Butyl steht.

Gruppe Ib: Diejenigen Verbindungen innerhalb der Untergruppe Ia, worin $R_1$ Wasserstoff bedeutet.

Gruppe Ic: Verbindungen der Formel I innerhalb der Untergruppe Ia, worin $R_1$ die Gruppe

-Si($R_4$)($R_5$)($R_6$) repräsentiert, wobei $R_4$, $R_5$ und $R_6$ unabhängig voneinander für $C_1$-$C_4$-Alkyl, Benzyl oder Phenyl stehen; und $R_2$ für Methyl, Ethyl, Isopropyl oder sek.-Butyl steht.

Gruppe Id: Diejenigen Verbindungen innerhalb der Untergruppe Ic, worin $R_1$ für Trimethylsilyl, tris(tert.-Butyl)-silyl, Diphenyl-tert.-butylsilyl, bis(Isopropyl)methylsilyl, Triphenylsilyl, Dimethyl-(2,3-dimethyl-2-butyl)silyl oder tert.-Butyl-dimethylsilyl bedeutet; und $R_2$ für Methyl, Ethyl, Isopropyl oder sek.-Butyl steht.

Gruppe Ie: Diejenigen Verbindungen im Umfang der Untergruppe Ia, worin $R_1$ eine Acylgruppe bedeutet.

Gruppe If: Diejenigen Verbindungen im Umfang der Untergruppe Ia, worin $R_1$ eine Acetyl- oder Benzoylgruppe bedeutet.

Gruppe Ig: Diejenigen Verbindungen im Umfang der Untergruppe Ia, worin R für einen Rest ausgewählt aus der Reihe 3-Methyl-3-oxetanyl, 3-Ethyl-3-oxetanyl, 3-Pentyl-3-oxetanyl, 2-Tetrahydrofuranyl, 3-Tetrahydrofuranyl, 2-Methyl-2-tetrahydrofuranyl, 2-Ethyl-2-tetrahydrofuranyl, 2-Pentyl-2-tetrahydrofuranyl, 2-Phenyl-2-tetrahydrofuranyl, 2,3-Dimethyl-2-tetrahydrofuranyl, 2,5-Dimethyl-2-tetrahydrofuranyl, 3-Ethyl-2-methyl-2-tetrahydrofuranyl, 2-Tetrahydropyranyl, 3-Tetrahydropyranyl, 2-Methyl-2-tetrahydropyranyl, 2-Ethyl-2-tetrahydropyranyl, 2-Propyl-2-tetrahydropyranyl, 2-Butyl-2-tetrahydropyranyl, 2-Pentyl-2-tetrahydropyranyl, 2-Phenyl-2-tetrahydropyranyl, 2,6-Dimethyl-2-tetrahydropyranyl, 2,6,6-Trimethyl-2-tetrahydropyranyl, 3,4-Dihydro-2H-pyranyl; und $R_1$ und $R_2$ die oben angegebene Bedeutung haben.

Besonders bevorzugte Einzelsubstanzen der Formel I sind:

13β-(3′-Methyl-3′-oxetancarbonyloxy)-milbemycin $A_4$;
13β-(3′-Methyl-3′-oxetancarbonyloxy)-milbemycin D;
13β-(3′,4′-Dihydro-2H-pyran-2′-carbonyloxy)-milbemycin $A_4$;
13β-(3′-Methyl-2′-dihydrobenzopyranyloxy)-milbemycin $A_4$;

Die vorliegende Erfindung betrifft nicht nur die Verbindungen der Formel I, sondern auch Verfahren, die es erlauben, in der 13-Position von Milbemycin- oder 13-Deoxy-22,23-dihydro-Avermectinaglykon-Derivaten eine β-Acyloxy-Gruppe gezielt einzuführen und damit zu den hochwirksamen neuen Parasitiziden und Insektiziden der Formel I zu gelangen, die gleichzeitig auch für weitere Derivatisierungen verwendet werden können.

Zur Herstellung von Estern der Formel I, worin RCOO- in der β-Position steht, geht man von einer Verbindung der Formel II

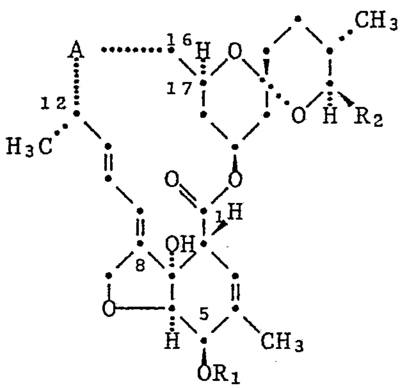

(II)

worin A für eine der Gruppen a oder b

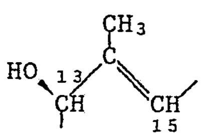

oder

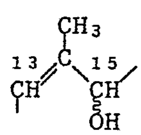

(a) (b)

[= 13β-Hydroxy-$\Delta^{14,15}$] [= $\Delta^{13,14}$-15-hydroxy]

steht, worin $R_1$ und $R_2$ die für Formel I angegebenen Bedeutungen haben, aus.

Eine Verbindung der Formel II, in welcher $R_1$ eine Schutzgruppe bedeutet und $R_2$ die unter Formel I angegebenen Bedeutungen besitzt, wird mit einem zur Einführung einer 13β-Estergruppe geeigneten Reagenz behandelt. Danach kann die $R_1$-Schutzgruppe, sofern eine freie 5-Hydroxy-Verbindung erwünscht ist, hydrolytisch abgespalten werden.

Verbindungen der Formel II, worin A für die Gruppe a steht, werden hier und im folgenden mit IIa und die Verbindungen mit der Gruppe b mit IIb bezeichnet.

Zur Einführung der 13β-Estergruppe in Verbindungen der Formel II geeignete Reagenzien sind beispielsweise:

a) Säuren der Formel III
RCOOH (III)
b) Säurehalogenide der Formel IV
RCOhal (IV),
worin hal Halogen, bevorzugt Chlor oder Brom, bedeutet,
c) Säureanhydride der Formel V
$(RCO)_2O$ (V),
wobei sich die Säuren für die Herstellung aller Verbindungen der Formel II eignen, vorzugsweise aber für Verbindungen der Formel IIb verwendet werden, während Säurehalogenide und Säureanhydride bei Verbindungen der Formel IIa zum Einsatz gelangen.

In den vorstehend angegebenen Formeln III-V besitzt R die unter Formel I angegebenen Bedeutungen.

Die Reaktionen zur Herstellung von Verbindungen der Formel I werden vorzugsweise mit Verbindungen der Formeln IIa oder IIb durchgeführt, deren reaktive 5-Hydroxy-Gruppe geschützt ist.

Verbindungen der Formel I, worin $R_1$ eine Schutzgruppe darstellt, lassen sich durch einfache, beispielsweise hydrolytische Abspaltung der Schutzfunktion in die hochaktiven freien 5-Hydroxy-derivate ($R_1 = H$) überführen und haben somit Zwischenprodukte-Charakter. Im übrigen wird der biologische Wert dieser Verbindungen durch die Schutzgruppe nicht gemindert.

Das Verfahren wird im allgemeinen in einem reaktions-inerten Lösungsmittel oder in einem der beteiligten Reaktanden, sofern diese flüssig sind, durchgeführt. Geeignete Lösungsmittel sind bei spielsweise: Ether und etherartige Verbindungen wie Dialkylether (Diethylether, Diisopropylether, tert.-Butylmethylether, Dimethoxyethan, Dioxan, Tetrahydrofuran oder Anisol); halogenierte Kohlenwasserstoffe wie Chlorbenzol, Methylenchlorid, Ethylenchlorid, Chloroform, Tetrachlorkohlenstoff oder Tetrachlorethylen; oder Sulfoxyde wie Dimethylsulfoxyd; ferner aromatische oder aliphatische Kohlenwasserstoffe, wie Benzol, Toluol, Xylole, Petrolether, Ligroin oder Cyclohexan. In manchen Fällen kann es von Vorteil sein, wenn die Reaktionen unter Schutzgasatmosphäre (beispielsweise Argon, Helium oder Stickstoff) und/oder in absoluten Lösungsmitteln durchgeführt werden. Gewünschtenfalls können die Endprodukte auf übliche Art und Weise gereinigt werden, beispielsweise durch Waschen, Digerieren, Extraktion, Umkristallisation oder Chromatographie.

Die Umsetzung von Verbindungen der Formel IIa oder IIb mit Säuren der Formeln III erfolgt in Gegenwart von Orthoestern sowie in Gegenwart katalytischer Mengen einer weiteren Säure. Als dafür geeignete Säuren können Protonensäuren oder Lewis-Säuren eingesetzt werden. Beispiele derartiger Säuren sind anorganische Säuren: Halogenwasserstoffsäure wie Chlorwasserstoffsäure, Bromwasserstoffsäure oder Jodwasserstoffsäure, Perchlorsäure sowie Schwefelsäure und organische Säuren wie Essigsäure, Trifluoressigsäure, Trichloressigsäure, Propionsäure, Oxalsäure, Ameisensäure, Benzolsulfonsäure, p-Toluolsulfonsäure oder Methansulfonsäure sowie Lewis-Säuren, wie $BF_3$, $AlCl_3$ oder $ZnCl_2$. Besonders bevorzugt sind p-Toluolsulfonsäure (auch als TsOH bezeichnet) und Schwefelsäure.

Die bei dieser Reaktion benötigten Orthoester haben die Formel VI
$R_{10}C(OR_{11})_3$ (VI),
worin $R_{10}$ Wasserstoff oder $C_1$-$C_4$-Alkyl, bevorzugt Methyl, und $R_{11}$ $C_1$-$C_4$-Alkyl, bevorzugt Methyl oder Ethyl bedeuten.

Bei Verwendung von Säuren der Formeln III zur Herstellung von Verbindungen der Formel I liegen die Reaktionstemperaturen im allgemeinen im Bereich von 0° bis 150°C, bevorzugt von 20° bis 130°C.

Die Reaktion von Verbindungen der Formel IIa mit Säurehalogeniden oder Säureanhydriden der Formeln IV bzw. V wird normalerweise in den obengenannten reaktionsinerten Lösungsmitteln im allgemeinen bei Temperaturen von -20° bis 100°C, vorzugsweise von 0° bis 70°C, durchgeführt. Zum Abfangen der dabei als Nebenprodukt gebildeten Säuren findet die Reaktion zweckmässigerweise in Gegenwart eines Neutralisationsmittels statt.

Als solche kommen organische Basen in Betracht, beispielsweise tertiäre Amine wie Trialkylamine (Trimethylamin, Triethylamin, Diisopropylmethylamin oder Tripropylamin), Pyridin und Pyridinbasen (4-Dimethylaminopyridin oder 4-Pyrrolidylaminopyridin), bevorzugt ist Pyridin. Das Neutralisationsmittel wird üblicherweise in mindestens äquimolarer Menge in bezug auf die Ausgangsstoffe eingesetzt.

Bei der Reaktion von Verbindungen der Formel IIb mit Säuren der Formeln III in Gegenwart von Orthoestern der Formel VI sowie einer katalytisch wirksamen Säure können neben den Verbindungen der Formel I als Nebenprodukte auch Verbindungen der Formel VII

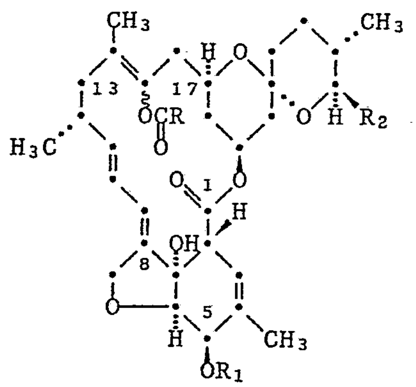

(VII)

worin $R_1$, $R_2$ und R die unter Formel I angegebenen Bedeutungen besitzen, gebildet werden.

Die erhaltenen Reaktionsprodukte lassen sich durch übliche Trennungsmethoden, wie beispielsweise durch fraktionierte Kristallisation oder durch Chromatographie, voneinander trennen. Unter Chromatographie werden Säulen-, Dickschicht- oder Dünnschicht-Chromatographie sowie bevorzugt Hochdruck-Flüssigkeits-Chromatographie an mineralischen Trägermaterialien wie Silicagel oder an organischen Austauscherharzen verstanden.

In einer anderen Verfahrensvariante setzt man Säuren der Formel III als solche ein und führt die Reaktion in Gegenwart wasserabspaltender Reagenzien durch. Beispielsweise arbeitet man in Anwesenheit von Dicyclohexylcarbodiimid und Pyridin oder in Gegenwart von Azodicarbonsäuredialkylester und Triphenylphosphin.

Die zur Gewinnung der erfindungsgemässen Verbindungen der Formel I mittels der hierin beschriebenen Verfahren benötigten Oxacycloalkancarbonsäuren, deren Säurehalogenide bzw. deren Säureanhydride sind bekannt (vgl. z.B. Bull. Soc. Chim. Fr. 1968, 344 - 351; Justus Liebigs Ann. Chem. 1973, 365 - 374; Chim. Ind. 1978, 60, 16 - 17; J. Org. Chem. 1982, 47, 2364-9.) oder lassen sich analog zu den bekannten Vertretern herstellen.

Die Verbindungen der Formel IIb [= $\Delta^{13,14}$-15-Hydroxy], worin $R_1$ und $R_2$ die für die Formel I genannten Bedeutung haben sind aus der Europ. Offenlegungsschrift EP 0147 852 bekannt geworden.

Die 13β-Hydroxy-$\Delta^{14,15}$-Verbindungen der Formel IIa lassen sich aus Verbindungen der Formel IIb, worin $R_1$ für eine Schutzgruppe steht, durch Reaktion mit Pyridiniumdichromat [= $(Pyr)_2$+$Cr_2O_7$] herstellen. Man arbeitet hierbei in Dimethylformamid und bei Temperaturen zwischen ca. -10° und +60°C.

Die $R_1$-Schutzgruppe wird, sofern gewünscht, anschliessend hydrolytisch abgespalten.

Die Entfernung dieser Silyl- und Acylreste $R_1$ in der 5-Position geschieht durch selektive milde Hydrolyse (→ $R_1$=H) beispielsweise mit Arylsulfonsäure in alkoholischer Lösung, mit einer wässrigen HF-Lösung in Acetonitril im Temperaturbereich -30° bis +30°C, mit Pyridiniumfluorid in Pyridin/THF oder nach einer anderen dem Fachmann geläufigen Methode.

Das beschriebene Verfahren zur Herstellung der Verbindungen der Formel I ist in allen Teilschritten ein Bestandteil vorliegender Erfindung.

Die Verbindungen der Formel I eignen sich ausgezeichnet zur Bekämpfung von Schädlingen an Tieren und Pflanzen, darunter tierparasitären Ekto-Parasiten. Zu letzteren zählen unter der Ordnung Acarina insbesondere Schädlinge der Familien Ixodidae, Dermanyssidae, Sarcoptidae, Psoroptidae; die Ordnungen Mallophaga; Siphonaptera, Anoplura (z.B. Familie der Haemotopinidae); unter der Ordnung Diptera insbesondere Schädlinge der Familien Muscidae, Calliphoridae, Oestridae, Tabanidae, Hippoboscidae, Gastrophilidae.

Die Verbindungen I sind auch einsetzbar gegen Hygiene-Schädlinge, insbesondere der Ordnungen Diptera mit den Familien Sarcophagidae, Anophilidae, Culicidae; der Ordnung Orthoptera, der Ordnung Dictyoptera (z.B. Familie Blattidae) und der Ordnung Hymenoptera (z.B. Familie Formicidae).

Die Verbindungen I besitzen auch nachhaltige Wirksamkeit bei pflanzenparasitären Milben und Insekten. Bei Spinnmilben der Ordnung Acarina sind sie wirksam gegen Eier, Nymphen und Adulte von Tetranychidae (Tetranychus spp. und Panonychus spp.).

Hohe Aktivität besitzen sie bei den saugenden Insekten der Ordnung Homoptera, insbesondere gegen Schädlinge der Familien Aphididae, Delphacidae, Cicadellidae, Psyllidae, Loccidae, Diaspididae und Eriophydidae (z.B. die Rostmilbe auf Citrusfrüchten): Der Ordnungen Hemiptera; Heteroptera und Thysanoptera; sowie bei den pflanzenfressenden Insekten der Ordnungen Lepidoptera; Coleoptera; Diptera und Orthoptera.

Sie sind ebenfalls als Bodeninsektizide und Bodennematizide gegen Schädlinge im Erdboden sowie im Wurzelbereich von Pflanzen geeignet.

Die Verbindungen der Formel I sind daher gegen alle Entwicklungsstadien saugender und fressender Insekten an Kulturen wie Getreide, Baumwolle, Reis, Mais, Soja, Kartoffeln, Gemüse, Früchten, Tabak, Hopfen, Citrus, Avocados und anderen wirksam.

Die Verbindungen der Formel I sind auch wirksam gegen Pflanzen-Nematoden der Arten Meloidogyne, Heterodera, Pratylenchus, Ditylenchus, Radopholus, Rizoglyphus und andere.

Besonders aber sind die Verbindungen gegen Helminthen wirksam, unter denen die endoparasitären Nematoden die Ursache schwerer Erkrankungen an Säugetieren und Geflügel sein können, z.B. an Schafen, Schweinen, Ziegen, Rindern, Pferden, Eseln, Hunden, Katzen, Meerschweinchen, Ziervögeln. Typische Nematoden dieser Indikation sind: Haemonchus, Trichostrongylus, Ostertagia, Nematodirus, Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris, Ancylostoma, Uncinaria, Toxascaris und Parascaris. Der besondere Vorteil der Verbindungen der Formel I ist ihre Wirksamkeit gegen solche Parasiten, die gegen Wirkstoffe auf Benzimidazol-Basis resistent sind.

Gewisse Spezies der Arten Nematodirus, Cooperia und Oesophagostomum greifen den Intestinaltrakt des Wirtstiers an, während andere der Arten Haemonchus und Ostertagia im Magen und solche der Art Dictyo caulus im Lungengewebe parasitieren. Parasiten der Familien Filariidae und Setariidae finden sich im internen Zellgewebe und den Organen, z.B. dem Herzen, den Blutgefässen, den Lymphgefässen und dem subcutanen Gewebe. Hier ist vor allem der Herzwurm des Hundes, Dirofilaria immitis, zu nennen. Die Verbindungen der Formel I sind gegen diese Parasiten hoch wirksam.

Sie sind ferner zur Bekämpfung von humanpathogenen Parasiten geeignet, unter denen als typische, im Verdauungstrakt vorkommende Vertreter solche der Arten Ancylostoma, Necator, Ascaris, Strongyloides, Trichinella, Capillaria, Trichuris und Enterobius zu nennen sind. Wirksam sind die Verbindungen vorliegender Erfindung auch gegen Parasiten der Arten Wuchereria, Brugia, Onchocerca und Loa aus der Familie der Filariidae, die im Blut, im Gewebe und verschiedenen Organen vorkommen, ferner gegen Dracunculus und Parasiten der Arten Strongyloides und Trichinella, die speziell den Gastro-Intestinalkanal infizieren.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Verbindungen der Formel I werden bei Warmblütern in Aufwandmengen von 0,01 bis 10 mg/kg Körpergewicht angewendet, über geschlossenen Kultur-Anbauflächen, in Pferchen, Stallungen oder sonstigen Räumen in Mengen von 10 g bis 1000 g pro Hektar.

Die Formulierungen, d.h. die den Wirkstoff der Formel I enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22

C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Die in der Formulierungstechnik gebräuchlichen Tenside sind u.a. in folgender Publikation beschrieben: "Mc Cutcheon's Detergents and Emulsifiers Annual" MC Publishing Corp., Ridgewood, New Jersey, 1982.

Die pestiziden Zubereitungen enthalten in der Regel 0,01 bis 95 %, insebesondere 0,1 bis 80 %, Wirkstoff der Formel I, 5 bis 99,99 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel mit 1-10'000 ppm Wirkstoffgehalt.

Ein weiterer Gegenstand vorliegender Erfindung betrifft daher Schädlingsbekämpfungsmittel, die neben üblichen Trägerstoffen und/oder Verteilungsmitteln als mindestens einen Wirkstoff eine Verbindung der Formel I enthalten.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

## Herstellungsbeispiele

## Herstellung von Ausgangs- und Zwischenprodukten

### Beispiel A1: Herstellung von 5-O-tert.Butyldimethylsilyl-13β-hydroxy-milbemycin D und von 13β-Hydroxy-milbemycin D (Formel Ia)

Eine Lösung bestehend aus 286 mg (0,41 mmol) 5-O-tert.Butyldimethylsilyl-15-hydroxy-$\Delta^{13,14}$-milbemycin D und 209 mg (0,56 mmol) Pyridiniumdichromat (PDC) in 3 ml Dimethylformamid (DMF) wird 30 min bei Raumtemperatur gerührt. Anschliessend wird 1 ml Isopropanol zugegeben, 5 min weitergerührt und dann mit 50 ml Ether verdünnt. Nach weiteren 10 min wird das Gemisch durch Kieselgel filtriert und eingeengt. Bei der Chromatographie des Rohproduktes an 20 g Kieselgel (Ether/Hexan 1:2) werden 165 mg (57 %) 5-O-t-Butyldimethylsilyl-13β-hydroxy-Milbemycin D erhalten.

$^{1}$H-NMR (300 MHz; $CDCl_3$; TMS):
1,59 ppm (br. s) ($C_{14}CH_3$)
3,70 ppm (d; J = 10 Hz) ($C_{13}H$).

105 mg (0,153 mmol) der so gewonnenen Verbindung werden mit 1 ml einer 1 %igen Lösung von p-Toluolsulfonsäure in Methanol 1 Stunde bei Raumtemperatur gerührt. Das Gemisch wird mit 20 ml Ether verdünnt, durch Kieselgel filtriert, eingeengt, und der Rückstand wird an ca. 10 g Kieselgel chromatographiert (Aceton/Dichlormethan 1:4), wobei 73 mg (83 %) 13β-Hydroxy-Milbemycin D erhalten werden.

$^{1}$H-NMR (300 MHz; $CDCl_3$; TMS):
1,58 ppm (br.s)($C_{14}CH_3$)
3,71 ppm (d; J = 10 Hz)($C_{13}H$).

### Beispiel A2: Herstellung von 5-O-tert.Butyldimethylsilyl-13β-hydroxy-milbemycin $A_4$

Eine Lösung bestehend aus 1,06 mg (1,59 mmol) 5-O-tert.Butyldimethylsilyl-15-hydroxy-$\Delta^{13,14}$-milbemycin $A_4$ und 383 mg (1,02 mmol) Pyridiniumdichromat (PDC) in 5 ml Dimethylformamid (DMF) wird 30 min. bei Raumtemperatur gerührt. Anschliessend wird 1 ml Isopropanol zugegeben, 5 min. weitergerührt und dann mit 50 ml Ether verdünnt. Nach weiteren 10 min. wird das Gemisch durch Kieselgel filtriert und eingeengt. Nach chromatographischer Reinigung des Rohproduktes an 20 g Kieselgel (Ether/Hexan 1:2) werden 625 mg (59 %) 5-O-Butyldimethylsilyl-13β-hydroxy-Milbemycin A4 erhalten.

$^{1}$H-NMR (300 MHz; $CDCl_3$; TMS):
0,98 ppm (t; J = 7 Hz) ($CH_3CH_2$)
1,95 ppm (br. s) ($C_{14}CH_3$)
3,69 ppm (d; J = 9 Hz) ($C_{13}H$).

## Herstellung von Endprodukten der Formel I:

### Beispiel H1: Herstellung von 13β-(13'-Methyl-3'-oxetancarbonyloxy)-milbemycin $A_4$

Zu einer Lösung von 1.54 g (2.29 mmol) 5-O-tert.-Butyldimethylsilyl-13β-hydroxy-milbemycin $A_4$ in 10 ml trockenem Chloroform werden 2,5 ml abs. Pyridin, eine katalytische Menge 4-N,N-Dimethylaminopyridin und 440 mg (3.28 mmol) 3-Methyl-3-oxetancarbonsäurechlorid zugegeben, und das Gemisch wird unter Argon 20 h bei Raumtemperatur gerührt. Die Aufarbeitung in Diethylether und die chromatographische Reinigung an 300 g Kieselgel (Laufmittel Essigsäureethylester/Hexan 1:4) ergibt 1,26 g (71 %) 5-O-tert.-Butyldimethylsilyl-13β-(3'-methyl-3'-oxetancarbonyloxy)-milbemycin $A_4$. Die Behandlung von 1,23 g (1,60 mmol) dieses Materials mit 15 ml einer 1 % Lösung von p-TsOH in MeOH während 2 Std. bei 0°C ergibt nach der Aufarbeitung in Diethylether und der chromatographischen Reinigung an 150 g Kieselgel (Laufmittel Essigsäureethylester/Hexan 1:1).

0,855 g (81 %) 13β-(3'-Methyl-3'-oxetancarbonyloxy)-milbemycin $A_4$

$^1$H-NMR (300 MHz, $CDCl_3$, TMS):
1,55 ppm (s) ($C_{14}CH_3$)

1,60 ppm (s) ($CH_2OCH_2C(\underline{CH_3})CO$)

4.38 - 4.42 und 4.90 - 4.94 ppm
(2 m) ($\underline{CH_2}O\underline{CH_2}C(CH_3)CO$)

5.00 ppm (d, J = 10.5 Hz) ($C_{13}H$)
MS (FD) m/e: 656 (M+, $C_{37}H_{52}O_{10}$)

Beispiel H2: Herstellung von 13β-(3′-Methyl-3′-oxetancarbonyloxy)-milbemycin D

Eine Lösung von 200 mg (0,29 mmol) 5-O-tert.-Butyldimethylsilyl-15-hydroxy-$\Delta^{13,14}$-milbemycin D, 132 mg (1,14 mmol) 3-Methyl-3-oxetancarbonsäure und 94 mg (0,58 mmol) Essigsäuretriethyl-orthoester in 6 ml abs. Toluol wird in einem Kolben mit Clarsen-Aufsatz während 6 min. auf 100°C erhitzt. Die Aufarbeitung in Diethylether und chromatographische Reinigung an 120 g Kieselgel (Laufmittel Diethylether/Hexan 1:2) ergibt 93 mg (41 %) 5-O-tert.-Butyldimethylsilyl-13β-(3′-methyl-3′-oxetancarbonyloxy)milbemycin D und 53 mg (23 %) 5-O-tert.-Butyldimethylsilyl-15-(3′-methyl-3′-oxetancarbonyloxy)-$\Delta^{13,14}$ milbemycin D. 92 mg (0,117 mmol) 5-O-tert.-Butyldimethylsilyl-13β-(3′-methyl-3′-oxetancarbonyloxy)-milbemycin D werden während 1.5 Std. bei Raumtemperatur mit 6 ml einer Lösung von 1 % p-TsOH in Methanol behandelt, wobei nach der Aufarbeitung, und der chromatographischen Reinigung an 70 mg Kieselgel (Laufmittel Diethylether) 67 mg (85 %) 13β-(3′-Methyl-3′-oxetancarbonyloxy)-milbemycin D erhalten.

$^1$H-NMR (300 MHz, $CDCl_3$, TMS):
1,55 ppm (br.s) ($C_{14}CH_3$)

1,60 ppm (s) ($CH_2OCH_2C(\underline{CH_3})CO$)

4.38 - 4.42 und 4.89 - 4.94 ppm
(2 m) ($\underline{CH_2}O\underline{CH_2}C(CH_3)CO$)

5.00 ppm (d, J = 10.5 Hz) ($C_{13}H$)

Beispiel H3: Herstellung von 13β-(3′,4′-Dihydro-2H-pyran-2′-carbonyloxy)-milbemycin $A_4$

Zu einer Lösung von 134 mg (0,30 mmol) 5-O-tert.-Butyldimethylsilyl-13β-hydroxy-milbemycin $A_4$ in 2 ml trockenem Dichlormethan werden 32µl abs. Pyridin und 1,2 mg 4-N,N-Dimethylaminopyridin zugegeben. Bei 0° werden unter Argon eine Lösung von 3,4-Dihydro-2H-pyran-2- carbonsäurechlorid in Benzol [hergestellt in situ aus 75 mg (0,50 mmol) 3.4-Dihydro-2H-pyran-2-carbonsäure-Natriumsalz in 2 ml abs. Benzol, 2 Tropfen N,N-Dimethylformamid und 34 µl (0,40 mmol) Oxalylchlorid; 2 Std. Raumtemperatur] zugetropft und das Gemisch 17 h bei 0 - 5°C und anschliessend 6 h bei Raumtemperatur gerührt. Die Aufarbeitung in Diethylether und die chromatographische Reinigung an 70 g Kieselgel (Laufmittel Diethylether/Hexan 1:5) ergibt 123 mg (78 %) 5-O-tert.-Butyldimethylsilyl-13β-(3,4-dihydro-2H-pyran-2-carbonyloxy)-milbemycin $A_4$. 60 mg (0,076 mmol) dieses Materials werden in 4 ml einer Lösung von 10 % HF/Pyridin-Komplex-reagenz in THF während 1h 15min bei 60°C gerührt. Die Aufarbeitung in Diethylether und die chromatographische Reinigung an 65 g Kieselgel (Laufmittel Essigsäureethylester/Hexan 1:1) ergibt 42 mg (82 %) 13β-(3′-4′-Dihydro-2H-pyran-2′-carbonyloxy)-milbemycin $A_4$.

$^1$H-NMR (300 MHz, $CDCl_3$, TMS):
1,54 ppm (br. s) ($C_{14}CH_3$)
1,85 pppm (br. s) ($C_4CH_3$)
5,04 ppm (d, J = 10 Hz) ($C_{13}H$)
6,38 ppm (d, J = 6 Hz) (C ,H)
MS (FD) m/e: 668 (M+, $C_{38}H_{52}O_{10}$).

Analog zu den beschriebenen Arbeitsweisen lassen sich auch die nachfolgend genannten Vertreter der Formel I herstellen.

Beispiel H4: 13β-(3′-Methyl-2′-dihydrobenzopyranylcarbonyloxy)-milbemycin $A_4$

$^1$H-NMR (300 MHz; $CDCl_3$; TMS):
5,04 und 5.07 ppm (2d, J = 10 Hz) ($C_{13}H$)
6,8-7,2 ppm (m) (4 aromat. H).

Tabelle 1

Typische Vertreter von Verbindungen der Formel I, worin R für die Gruppe

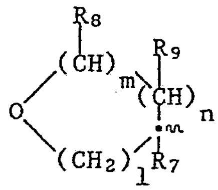

$R_1$ für Wasserstoff steht und $R_2$ die oben angegebene Bedeutung hat, sind:

| Verb. Nr. | R | $R_2$ | Herst. beisp. |
|---|---|---|---|
| 1.1<br>1.2<br>1.3<br>1.4 | 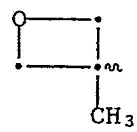 | $CH_3$<br>$C_2H_5$<br>iso-$C_3H_7$<br>sec.-$C_4H_9$ | H1<br>H2 |
| 1.5<br>1.6<br>1.7<br>1.8 | 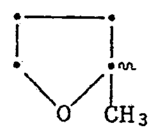 | $CH_3$<br>$C_2H_5$<br>iso-$C_3H_7$<br>sec.-$C_4H_9$ | |
| 1.9<br>1.10<br>1.11<br>1.12 | 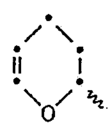 | $CH_3$<br>$C_2H_5$<br>iso-$C_3H_7$<br>sec.-$C_4H_9$ | H3 |
| 1.13<br>1.14<br>1.15<br>1.16 | 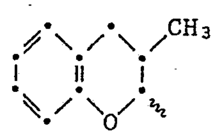 | $CH_3$<br>$C_2H_5$<br>iso-$C_3H_7$<br>sec.-$C_4H_9$ | H4 |
| 1.17<br>1.18<br>1.19<br>1.20 | 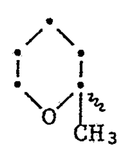 | $CH_3$<br>$C_2H_5$<br>iso-$C_3H_7$<br>sec.-$C_4H_9$ | |

Formulierungsbeispiele für den Wirkstoff der Formel I

(% = Gewichtsprozent)

| Spritzpulver | a) | b) | c) |
|---|---|---|---|
| Wirkstoff aus den Tabellen | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyethylenglykol-ether (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| Emulsions-Konzentrat | |
|---|---|
| Wirkstoff aus den Tabellen | 10 % |
| Octylphenolpolyethylenglykolether (4-5 Mol AeO) | 3 % |
| Ca-Dodecylbenzolsulfonat | 3 % |
| Ricinusölpolyglykolether (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Stäubemittel | a) | b) |
|---|---|---|
| Wirkstoff aus den Tabellen | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

Extruder Granulat

Wirkstoff aus den Tabellen 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert und anschliessend im Luftstrom getrocknet.

Tabellen bzw. Boli

I Ein Wirkstoff aus den Tabellen 33,0 %
Methylcellulose 0,80 %
Kieselsäure hochdispers 0,80 %
Maisstärke 8,40 %

Methylcellulose in Wasser einrühren und quellen lassen; Kieselsäure in die Quellung einrühren und homogen suspendieren. Wirkstoff und Maisstärke mischen. In dieses Mischung die wässrige Suspension einarbeiten und zu einem Teig kneten. Diese Masse durch ein Sieb (Maschenweite 12 M) granulieren und dann trocknen.

II Milchzucker krist. 22,50 %
Maisstärke 17,00 %
mikrokrist. Cellulose 16,50 %
Magnesiumstearat 1,00 %

Alle 4 Hilfsstoffe gut mischen

Phasen I und II mischen und zu Tabletten oder Boli verpressen.

Falls die Verbindungen der Formel I bzw. entsprechende Mittel zur Bekämpfung von endoparasitären Nematoden, Cestoden und Trematoden bei Haus- und Nutztieren, wie Rindern, Schafen, Ziegen, Katzen und Hunden, verwendet werden, können sie den Tieren sowohl als Einzeldosis wie auch wiederholt verabreicht werden, wobei die einzelnen Gaben je nach Tierart verzugsweise zwischen 0,1 und 10 mg pro kg Körpergewicht betragen. Durch eine protrahierte Verabreichung erzielt man in manchen Fällen eine bessere Wirkung oder man kann mit geringeren Gesamtdosen auskommen. Der Wirkstoff bzw. die ihn enthaltenden Mittel können auch dem Futter oder den Tränken zugesetzt werden. Das Fertigfutter enthält die Wirkstoffkombinationen vorzugsweise in einer Konzentration von 0,005 bis 0,1 Gew. %. Die Mittel können in Form von Lösungen, Emulsionen, Suspensionen, Pulver, Tabletten, Bolussen oder Kapseln peroral den Tieren verabreicht werden. Soweit die physikalischen und toxikologischen Eigenschaften von Lösungen oder Emulsionen dies zulassen, können die Verbindungen der Formel I bzw. die enthaltende Mittel an Tieren auch beispielsweise subcutan injiziert, intraruminal verabreicht oder mittels der Pour-on-Methode auf den Körper der Tiere appliziert werden. Ferner ist eine Verabreichung des Wirkstoffs an die Tiere auch durch Lecksteine (Salz) oder Molasse-Blöcke möglich.

Biologische Beispiele

B-1. Wirkung gegen $L_1$-Larven von Lucilia sericata

1 ml einer wässrigen Suspension der zu prüfenden Aktivsubstanz werden so mit 3 ml eines speziellen Larvenzuchtmediums bei ca. 50° C vermischt, dass ein Homogenisat von wahlweise 100 ppm oder 10 ppm Wirkstoffgehalt entsteht. In jede Reagenzglas-Probe werden ca. 30 Lucilia-Larven ($L_1$) eingesetzt. Nach 4 Tagen wird die Mortalitätsrate bestimmt. Alle Verbindungen der Formel I aus den Herstellungsbeispielen erzielten mit 100 ppm eine Wirkung von 100 %. Bei einer Verdünnung auf 10 ppm erzielten die Verbindungen Nr. 1.2, und 1.3 ebenfalls noch volle Wirkung.

B-2. Akarizide Wirkung gegen Boophilus microplus (Biarra-Stamm

Auf einer PVC-Platte wird waagrecht ein Klebstreifen so befestigt, das darauf 10 mit Blut vollgesogene Zecken-Weibchen von Boophilus microplus (Biarra-Stamm) nebeneinander in einer Reihe mit dem Rücken aufgeklebt werden können. Jeder Zecke wird mit einer Injektionsnadel 1 µl einer Flüssigkeit injiziert, die eine 1:1-Mischung von Polyethylenglykol und Aceton darstellt und in der eine bestimmte Wirkstoffmenge von wahlweise 1, 0,5 oder 0,1 µl pro Zecke gelöst ist. Kontrolltiere erhalten eine wirkstofffreie Injektion. Nach der Behandlung werden die Tiere unter Normalbedingungen in einem Insektarium bei ca. 28° C und 80 % relativer Luftfeuchtigkeit gehalten, bis die Eiablage erfolgt und die Larven aus den Eiern der Kontrolltiere geschlüpft sind.

Die Aktivität einer geprüften Substanz wird mit der $IR_{90}$ bestimmt, d.h. es wird jene Wirkstoffdosis ermittelt, bei der noch nach 30 Tagen 9 von 10 Zeckenweibchen (= 90 %) Eier ablegen, die nicht schlupffähig sind.

Die Verbindungen der Formeln I aus den Tabellen, erzielen eine $IR_{90}$ von 0,5 µg.

B-3. Versuch an mit Nematoden (Haemonchus concortus und Trichostrongylus colubriformis) infizierten Schafen

Der Wirkstoff wird als Suspension formuliert mit einer Magensonde oder durch Injektion in den Pansen

eines Schafes gegeben, das mit Haemonchus concortus und Trychostrongylus colubriformis künstlich infiziert worden ist. Pro Dosis werden 1 bis 3 Tiere verwendet. Jedes Schaf wird nur einmal mit einer einzigen Dosis behandelt, und zwar wahlweise mit 0,5 mg oder 0,2 mg/kg Körpergewicht. Die Evaluierung erfolgt durch Vergleich der Anzahl der vor und nach Behandlung in Kot der Schafe ausgeschiedenen Wurmeier.

Gleichzeitig und gleichartig infizierte aber unbehandelte Schafe dienen als Kontrolle. Schafe, die mit einer der Verbindungen der Formeln I bei 1 mg/kg behandelt wurden, zeigten im Vergleich zu unbehandelten, aber infizierten Vergleichsgruppen keinen Nematodenbefall (= komplette Reduktion der Wurmeier in Kot).

B-4. Larvizidwirkung gegen Aedes aegypti

Auf die Oberfläche von 150 ml Wasser, das sich in einem Behälter befindet, wird soviel einer 0,1 %igen acetonischen Lösung des Wirkstoffes pipettiert, dass Konzentrationen von wahlweise 10 ppm, 3,3 ppm und 1,6 ppm erhalten werden. Nach Verdunsten des Acetons wird der Behälter mit ca. 30-40 3 Tage alten Aedes-Larven beschickt. Nach 1, 2 und 5 Tagen wird die Mortalität geprüft.

Die Verbindungen der Formeln I aus den Tabellen, wie z.B. die Verbindung Nr. 1.3 bewirkte in diesem Test bei einer Konzentration von 1,6 ppm bereits nach einem Tag eine vollständige Abtötung sämtlicher Larven.

B-5. Milbizide Wirkung gegen Dermanyssus gallinae

2 bis 3 ml einer Testlösung (100, 10, 1 und 0,1 ppm Aktivsubstanz) werden in einen nach oben offenen Glasbehälter gegeben und ca. 200 Milben in unterschiedlichen Entwicklungsstadien in diesen Behälter eingesetzt. Der Glasbehälter wird mit einem Wattebausch verschlossen und 10 Minuten gleichmässig, bis zur vollständigen Benetzung der Milben geschüttelt. Danach wird der Behälter umgekehrt hingestellt, bis die überschüssige Testlösung von der Watte aufgesogen ist. Der Behälter wird erneut gekehrt und die behandelten Zecken zur Evaluierung der Wirksamkeit der Testsubstanzen drei Tage unter Laborbedingungen beobachtet, wobei die Mortalität als Massstab für Wirksamkeit herangezogen wird.

Verbindungen aus den Herstellungsbeispielen wie. z.B. die Verb. Nr. 1.2 und 1.3 zeigen bei 100 ppm eine Wirkung von 100 %.

## Patentansprüche

1. Verbindungen der Formel I

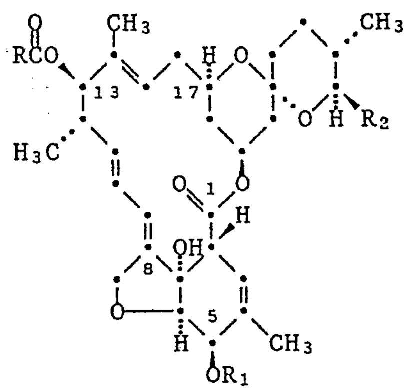

(I)

in welcher
$R_1$ Wasserstoff, oder eine OH-Schutzgruppe,
$R_2$ Methyl, Ethyl, Isopropyl oder sek.Butyl und
R für einen Oxacycloalkylrest steht.

2. Verbindungen der Formel I nach Anspruch 1, worin $R_1$ Wasserstoff, $R_3$-C(O)- oder $Si(R_4)(R_5)(R_6)$ bedeutet; $R_2$ für Methyl, Ethyl, Isopropyl oder sek. Butyl, R für einen Oxacycloalkyrest steht; $R_3$ für $C_1$-$C_6$-Alkyl, $C_1$-$C_6$-Haloalkyl oder unsubstituiertes oder durch Halogen, $C_1$-$C_3$-Alkyl, $CF_3$ oder Nitro substituiertes Phenyl steht und $R_4$, $R_5$ und $R_6$ unabhängig voneinander für $C_1$-$C_4$-Alkyl, Benzyl oder Phenyl stehen.

3. Verbindungen der Formel I nach Anspruch 2, worin $R_1$ bis $R_6$ die in Anspruch 2 angegebenen Bedeutungen haben und R für einen Oxacycloalkylrest steht, ausgewählt aus der Reihe 3-Methyl-3-oxetanyl, 3-Ethyl-3-oxetanyl, 3-Pentyl-3-oxetanyl, 2-Tetrahydrofuranyl, 3-Tetrahydrofuranyl, 2-Methyl-2-Tetrahydrofuranyl, 2-Ethyl-2-tetrahydrofuranyl, 2-Pentyl-2-tetrahydrofuranyl, 2-Phenyl-2-tetrahydrofuranyl, 2,3-Dimethyl-2-tetrahydrofuranyl, 2,5-Dimethyl-2-tetrahydrofuranyl, 3-Ethyl-2-methyl-2-tetrahydrofuranyl, 2-Tetrahydropyra nyl, 3-Tetrahydropyranyl, 2-Methyl-2-tetrahydropyranyl, 2-Ethyl-2-tetrahydropyranyl, 2-Propyl-2-tetrahydropyranyl, 2-Butyl-2-tetrahydropyranyl, 2-Pentyl-2-tetrahydropyranyl, 2-Phenyl-2-tetrahydropyranyl, 2,6-Dimethyl-2-tetrahydropyranyl, 2,6,6,-Trimethyl-2-tetrahydropyranyl und 3,4-Dihydro-2H-pyranyl.

4. Verbindungen der Formel I nach Anspruch 2, worin $R_1$ bis $R_6$ die in Anspruch 2 angegebenen Bedeutungen haben und R für einen Oxacycloalkylrest der Formel U steht

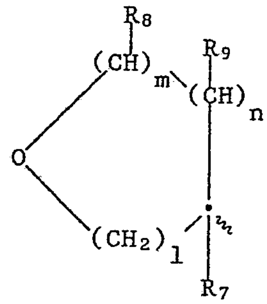

(U)

wobei 1, m und n unabhängig voneinander 0, 1, 2 oder 3 bedeuten, $R_7$, $R_8$ und $R_9$ unabhängig voneinander für Wasserstoff, Alkyl oder Phenyl stehen, gegebenenfalls auch eine endocyclische Doppelbindung vorliegt.

5. Verbindungen der Formel I nach Anspruch 4, worin R, $R_2$ bis $R_6$ die in Anspruch 4 angegebenen Bedeutungen haben und $R_1$ für Wasserstoff steht.

6. Verbindungen der Formel I nach Anspruch 4, worin R die in Anspruch 4 angegebene Bedeutung hat und $R_1$ die Gruppe $-Si(R_4)(R_5)(R_6)$ repräsentiert, wobei $R_4$, $R_5$ und $R_6$ unabhängig voneinander für $C_1$-$C_4$-Alkyl, Benzyl oder Phenyl stehen.

7. Verbindungen der Formel I nach Anspruch 6, worin R, $R_2$ bis $R_6$ die in Anspruch 6 angegebenen Bedeutungen haben und $R_1$ für Trimethylsilyl, tris(tert.-Butyl)-silyl, Diphenyl-tert.-butylsilyl, bis(Isopropyl)methylsilyl, Triphenylsilyl, Dimethyl-(2,3-dimethyl-2-butyl)silyl oder tert.-Butyl-dimethylsilyl bedeutet; und $R_2$ für Methyl, Ethyl, Isopropyl oder sek.-Butyl steht.

8. Verbindungen der Formel I nach Anspruch 4, worin R, $R_2$ bis $R_6$ die in Anspruch 4 angegebenen Bedeutungen haben und $R_1$ eine Acylgruppe repräsentiert.

9. Verbindungen der Formel I nach Anspruch 4, worin R, $R_2$ bis $R_6$ die in Anspruch 4 angegebenen Bedeutungen haben und $R_1$ eine Acetyl- oder Benzoylgruppe repräsentiert.

10. Eine Verbindung der Formel I nach Anspruch 1, ausgewählt aus der Reihe:
13β-(3′-Methyl-3′-oxetancarbonyloxy)-milbemycin $A_4$;
13β-(3′-Methyl-3′-oxetancarbonyloxy)-milbemycin D;
13β-(3′,4′-Dihydro-2H-pyran-2′-carbonyloxy)-milbemycin $A_4$;
13β-(3′-Methyl-2′-dihydrobenzopyranyloxy)-milbemycin $A_4$.

11. Verfahren zur Herstellung von Verbindungen der Formel I

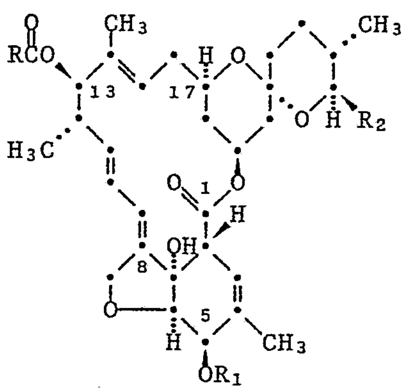

(I)

in welcher
$R_1$ Wasserstoff, oder eine OH-Schutzgruppe,
$R_2$ Methyl, Ethyl, Isopropyl oder sek.Butyl und
R für einen Oxacycloalkylrest steht,
dadurch gekennzeichnet, dass man eine Verbindung der Formel II

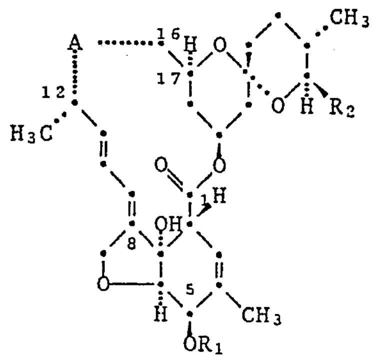

(II)

worin A für eine der Gruppen a oder b

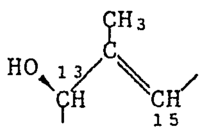

oder

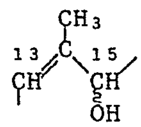

(a) (b)

[= 13β-Hydroxy-$\Delta^{14,15}$] [= $\Delta^{13,14}$-15-hydroxy]

steht, mit einem zur Einführung der 13β-Estergruppe geeigneten Reagenz umsetzt und anschliessend, falls erforderlich, die 5-OH-Schutzgruppe hydrolytisch abspaltet.

12. Verfahren nach Anspruch 11, dadurch gekennzeichnet, dass man als zur Einführung der 13β-Estergruppe geeignete Reagenzien

a) Säuren der Formel III

RCOOH (III)

b) Säurehalogenide der Formel IV

RCOhal (IV)

worin hal Halogen, bevorzugt Chlor oder Brom, bedeutet,

c) Säureanhydride der Formel V

$(RCO)_2O$ (V),

einsetzt, wobei R die unter Formel I angegebenen Bedeutungen hat.

13. Verfahren nach einem der Ansprüche 11 oder 12, dadurch gekennzeichnet, dass man in einem reaktionsinerten Lösungmittel arbeitet.

14. Verfahren nach Anspruch 12, dadurch gekennzeichnet, dass man die Reaktion

a) bei 0° bis 150° C, vorzugsweise 20° bis 130° C und Reaktion b) und c) bei -20° bis 100° C, vorzugsweise 0° bis 70° C durchführt.

15. Verfahren nach einem der Ansprüche 11 bis 14, dadurch gekennzeichnet, dass man in Gegenwart eines Neutralisationsmittels arbeitet.

16. Schädlingsbekämpfungsmittel zur Bekämpfung von Ekto-, Endoparasiten und Insekten, dadurch gekennzeichnet, dass es neben üblichen Trägerstoffen und Verteilungsmitteln eine Verbindung der Formel I enthält

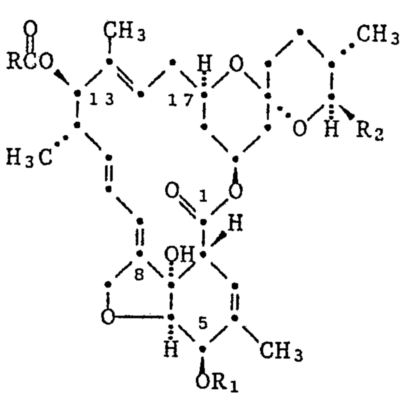

(I)

in welcher

$R_1$ Wasserstoff, oder eine OH-Schutzgruppe,
$R_2$ Methyl, Ethyl, Isopropyl oder sek.Butyl und
R für einen Oxacycloalkylrest steht.

17. Mittel nach Anspruch 16, dadurch gekennzeichnet, dass es eine Verbindung der Formel I nach einem der Ansprüche 2 bis 10 enthält.

18. Verwendung einer Verbindung der Formel I

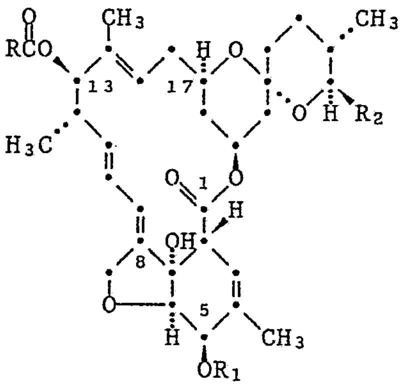

(I)

in welcher
$R_1$ Wasserstoff, oder eine OH-Schutzgruppe,
$R_2$ Methyl, Ethyl, Isopropyl oder sek.Butyl und
R für einen Oxacycloalkylrest steht,
zur Bekämpfung von Ekto-, Endoparasiten und Insekten, die Nutztiere oder Pflanzen befallen.

19. Verwendung nach Anspruch 18, zur Bekämpfung von Endoparasiten bei Nutztieren.

20. Verwendung nach Anspruch 19, gegen Nematoden.

21. Verwendung nach Anspruch 18 zur Bekämpfung von pflanzenpathogenen Nematoden.

22. Verfahren zur Bekämpfung von tier- oder pflanzenparasitären Schädlingen, dadurch gekennzeichnet, dass man eine parasitizid aktive Menge einer Verbindung der Formel I

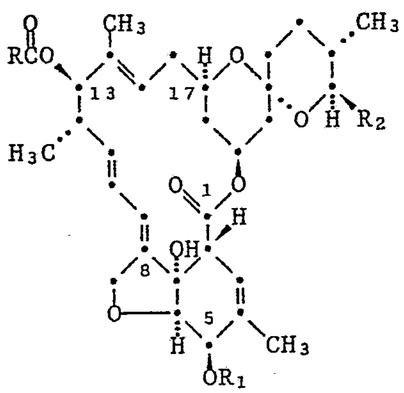

(I)

in welcher
$R_1$ Wasserstoff, oder eine OH-Schutzgruppe,
$R_2$ Methyl, Ethyl, Isopropyl oder sek.Butyl und
R für einen Oxacycloalkylrest steht,
an das Tier, die Pflanze oder den Lebensraum des Parasiten appliziert.

Europäisches Patentamt

# EUROPÄISCHER RECHERCHENBERICHT

Nummer der Anmeldung

EP 87 81 0370

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 184 308 (SANKYO)<br>* Anspruch 11; Seite 1, Zeilen 10-15 *<br>--- | 1,16 | C 07 D 493/22<br>C 07 F 7/08<br>A 01 N 43/90 //<br>(C 07 D 493/22<br>C 07 D 313:00<br>C 07 D 311:00<br>C 07 D 311:00<br>C 07 D 307:00 ) |
| Y | GB-A-2 168 345 (CIBA-GEIGY)<br>* Ansprüche 1,23 *<br>----- | 1,16 | |

| RECHERCHIERTE SACHGEBIETE (Int. Cl.4) |
|---|
| C 07 D 493/00<br>A 01 N 43/00<br>C 07 F 7/00 |

Der vorliegende Recherchenbericht wurde für alle Patentansprüche erstellt.

| Recherchenort | Abschlußdatum der Recherche | Prüfer |
|---|---|---|
| DEN HAAG | 12-10-1987 | ALFARO I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument